# EUROPEAN PATENT SPECIFICATION

(11) **EP 0 879 289 B1**
(45) Date of publication and mention of the grant of the patent: **05.07.2006**
(21) Application number: 97904103.5
(22) Date of filing: 03.02.1997
(51) Int. Cl.: C12N 15/55, C12N 9/22, C12N 5/10, A61K 38/46

(54) **HUMAN DNASE RESISTANT TO ACTIN INHIBITION**
MENSCHLICHE DNASE RESISTENT GEGEN ACTININHIBITOREN
DNASE HUMAINE RESISTANT A L'INHIBITION PAR L'ACTINE

(30) Priority: 05.02.1996 US 597078
(43) Date of publication of application: 25.11.1998
(73) Proprietor: GENENTECH, INC., South San Francisco, CA 94080-4990 (US)
(72) Inventor: BAKER, Kevin, P., San Mateo, CA 94402-1932 (US); BARON, Will, F., Brisbane, CA 94005 (US)
(74) Representative: Barz, Peter
(86) International application number: PCT/US1997/001506
(87) International publication number: WO 1997/028266

(56) References cited:
- WO-A-90/07572
- WO-A-93/25670
- HUM MOL GENET, SEP 1995, 4 (9) P1557-64, ENGLAND, XP002032854 PARRISH JE ET AL: "A muscle-specific DNase I-like gene in human Xq28."
- EMBL databank Accession number U56814 Rodriguez A. et al. 02-07-96 XP002032855

### SEQUENCE LISTING

(1) GENERAL INFORMATION:
   (i) APPLICANT: Genentech, Inc.
   (ii) TITLE OF INVENTION: HUMAN DNASE
   (iii) NUMBER OF SEQUENCES: 11
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Johnston, Sean A.
      (B) REGISTRATION NUMBER: 35,910
      (C) REFERENCE/DOCKET NUMBER: P1000PCT
   (ix) TELECOMMUNICATION INFORMATION:
      (A) TELEPHONE: 415/225-3562
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 1079 base pairs
      (B) TYPE: Nucleic Acid
      (C) STRANDEDNESS: Single
      (D) TOPOLOGY: Linear
   (xi) SEQUENCE DESCRIPTION: SEQ ID NO:1:
(2) INFORMATION FOR SEQ ID NO:3:
   (i) SEQUENCE CHARACTERISTICS:
      (A) LENGTH: 285 amino acids
      (B) TYPE: Amino Acid
      (D) TOPOLOGY: Linear
   (xi) SEQUENCE DESCRIPTION: SEQ ID NO:3:
   Met Arg Ile Cys Ser Phe Asn Val Arg Ser Phe Gly Glu Ser Lys
    1               5                  10                  15
   Gln Glu Asp Lys Asn Ala Met Asp Val Ile Val Lys Val Ile Lys
                   20                  25                  30
   Arg Cys Asp Ile Ile Leu Val Met Glu Ile Lys Asp Ser Asn Asn
                   35                  40                  45
   Arg Ile Cys Pro Ile Leu Met Glu Lys Leu Asn Arg Asn Ser Arg
                   50                  55                  60
   Arg Gly Ile Thr Tyr Asn Tyr Val Ile Ser Ser Arg Leu Gly Arg
                   65                  70                  75
   Asn Thr Tyr Lys Glu Gln Tyr Ala Phe Leu Tyr Lys Glu Lys Leu
                   80                  85                  90
   Val Ser Val Lys Arg Ser Tyr His Tyr His Asp Tyr Gln Asp Gly
                   95                  100                 105
   Asp Ala Asp Val Phe Ser Arg Glu Pro Phe Val Val Trp Phe Gln
                   110                 115                 120
   Ser Pro His Thr Ala Val Lys Asp Phe Val Ile Ile Pro Leu His
                   125                 130                 135
   Thr Thr Pro Glu Thr Ser Val Lys Glu Ile Asp Glu Leu Val Glu
                   140                 145                 150
   Val Tyr Thr Asp Val Lys His Arg Trp Lys Ala Glu Asn Phe Ile
                   155                 160                 165
   Phe Met Gly Asp Phe Asn Ala Gly Cys Ser Tyr Val Pro Lys Lys
                   170                 175                 180
   Ala Trp Lys Asn Ile Arg Leu Arg Thr Asp Pro Arg Phe Val Trp
                   185                 190                 195
   Leu Ile Gly Asp Gln Glu Asp Thr Thr Val Lys Lys Ser Thr Asn
                   200                 205                 210
   Cys Ala Tyr Asp Arg Ile Val Leu Arg Gly Gln Glu Ile Val Ser
                   215                 220                 225
   Ser Val Val Pro Lys Ser Asn Ser Val Phe Asp Phe Gln Lys Ala
                   230                 235                 240
   Tyr Lys Leu Thr Glu Glu Glu Ala Leu Asp Val Ser Asp His Phe
                   245                 250                 255
   Pro Val Glu Phe Lys Leu Gln Ser Ser Arg Ala Phe Thr Asn Ser
                   260                 265                 270
   Lys Lys Ser Val Thr Leu Arg Lys Lys Thr Lys Ser Lys Arg Ser
                   275                 280                 285
(2) INFORMATION FOR SEQ ID NO:4:
   (i) SEQUENCE CHARACTERISTICS:
      (A) LENGTH: 260 amino acids
      (B) TYPE: Amino Acid
      (D) TOPOLOGY: Linear
   (xi) SEQUENCE DESCRIPTION: SEQ ID NO:4:
   Leu Lys Ile Ala Ala Phe Asn Ile Gln Thr Phe Gly Glu Thr Lys
   1 5 10 15
   Met Ser Asn Ala Thr Leu Val Ser Tyr Ile Val Gln Ile Leu Ser
   20 25 30
   Arg Tyr Asp Ile Ala Leu Val Gln Glu Val Arg Asp Ser His Leu
   35 40 45
   Thr Ala Val Gly Lys Leu Leu Asp Asn Leu Asn Gln Asp Ala Pro
   50 55 60
   Asp Thr Tyr His Tyr Val Val Ser Glu Pro Leu Gly Arg Asn Ser
   65 70 75
   Tyr Lys Glu Arg Tyr Leu Phe Val Tyr Arg Pro Asp Gln Val Ser
   80 85 90
   Ala Val Asp Ser Tyr Tyr Tyr Asp Asp Gly Cys Glu Pro Cys Gly
   95 100 105
   Asn Asp Thr Phe Asn Arg Glu Pro Ala Ile Val Arg Phe Phe Ser
   110 115 120
   Arg Phe Thr Glu Val Arg Glu Phe Ala Ile Val Pro Leu His Ala
   125 130 135
   Ala Pro Gly Asp Ala Val Ala Glu Ile Asp Ala Leu Tyr Asp Val
   140 145 150
   Tyr Leu Asp Val Gln Glu Lys Trp Gly Leu Glu Asp Val Met Leu
   155 160 165
   Met Gly Asp Phe Asn Ala Gly Cys Ser Tyr Val Arg Pro Ser Gln
   170 175 180
   Trp Ser Ser Ile Arg Leu Trp Thr Ser Pro Thr Phe Gln Trp Leu
   185 190 195
   Ile Pro Asp Ser Ala Asp Thr Thr Ala Thr Pro Thr His Cys Ala
   200 205 210
   Tyr Asp Arg Ile Val Val Ala Gly Met Leu Leu Arg Gly Ala Val
   215 220 225
   Val Pro Asp Ser Ala Leu Pro Phe Asn Phe Gln Ala Ala Tyr Gly
   230 235 240
   Leu Ser Asp Gln Leu Ala Gln Ala Ile Ser Asp His Tyr Pro Val
   245 250 255
   Glu Val Met Leu Lys
   260
(2) INFORMATION FOR SEQ ID NO:5:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:5:
      ACTGTAGTTT AAATTCAACT GGAAAGTGGT CGCTGACATC CAGGG 45
(2) INFORMATION FOR SEQ ID NO:6:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:6:
      GATGTCATTG TGAAGGTCAT CAAACGCTGT GACATCATAC TCGTG 45
(2) INFORMATION FOR SEQ ID NO:7:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:7:
      GTGTTTTCCA GGGGAGCCCT TTGTGGTCTG GTTCCAATCT CCCCA 45
(2) INFORMATION FOR SEQ ID NO:8:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:8:
      CTGGAGGTCT CCCAGCACTG GCAGAGCAAG GACGTGATCC TGCTT 45
(2) INFORMATION FOR SEQ ID NO:9:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:9:
      GCCCAGCATC ATCGCGAAGT TCCTGGCTGG CTATCACCTC GCGCT 45
(2) INFORMATION FOR SEQ ID NO:10:
   (i) SEQUENCE CHARACTERISTICS:
      (A) LENGTH: 45 base pairs
      (B) TYPE: Nucleic Acid
      (C) STRANDEDNESS: Single
      (D) TOPOLOGY: Linear
   (xi) SEQUENCE DESCRIPTION: SEQ ID NO:10:
      CCAGTACAAG GAGATGTACC TCTTCGTTTA CAGGAAAGAC GCCGT 45
(2) INFORMATION FOR SEQ ID NO:11:
   (i) SEQUENCE CHARACTERISTICS:
      (A) LENGTH: 1124 base pairs
      (B) TYPE: Nucleic Acid
      (C) STRANDEDNESS: Single
      (D) TOPOLOGY: Linear
   (xi) SEQUENCE DESCRIPTION: SEQ ID NO:11:
   GAATTCCGGC CCATTACCTT CATTTCCTTG GGGATTGAAA CGCGTGATGG 50
   TGAGTTCCTC AGAGAAGTGA AAGTGACCTA GAGGGATCCA GTAATTCCTG 100
   TTATCAGCCT GCTTTATAAG TCAGTGAGCC AGGCACTGTC TTCATCCAGC 150
   CTGAAGTCCC AGGAGTGCAA AGATGTCCCT GCACCCAGCT TCCCCACGCC 200
   TGGCCTCCCT GCTGCTCTTC ATCCTTGCCC TCCATGACAC CCTGGCCCTA 250
   AGGCTCTGCT CCTTCAATGT GAGGTCCTTT GGAGCGAGCA AGAAGGAAAA 300
   CCATGAAGCC ATGGATATCA TTGTGAAGAT CATCAAACGC TGTGACCTTA 350
   TACTGTTGAT GGAAATCAAG GACAGCAGCA ACAACATCTG TCCCATGCTG 400
   ATGGAGAAGC TGAATGGAAA TTCACGAAGA AGCACAACAT ACAACTATGT 450
   GATTAGTTCT CGACTTGGAA GAAACACGTA CAAAGAGCAG TATGCCTTCG 500
   TCTACAAGGA GAAGCTGGTG TCTGTGAAGA CAAAATACCA CTACCATGAC 550
   TATCAGGATG GAGACACAGA CGTGTTTTCC AGGGAGCCCT TTGTGGTTTG 600
   GTTCCATTCC CCCTTTACTG CTGTCAAGGA CTTCGTGATT GTCCCCTTGC 650
   ACACAACTCC CGAGACCTCC GTTAAAGAGA TAGATGAGCT GGTCGATGTC 700
   TACACGGATG TGAGAAGCCA GTGGAAGACA GAGAATTTCA TCTTCATGGG 750
   TGATTTCAAC GCCGGCTGTA GCTATGTCCC CAAGAAGGCC TGGCAGAACA 800
   TTCGTTTGAG GACGGACCCC AAGTTTGTTT GGCTGATTGG GGACCAAGAG 850
   GACACTACGG TCAAGAAGAG TACCAGCTGT GCCTATGACA GGATTGTGCT 900
   TTGTGGACAA GAGATAGTCA ACTCCGTGGT TCCCCGTTCC AGTGGCGTCT 950
   TTGACTTTCA GAAAGCTTAT GACTTGTCTG AGGAGGAGGC CCTGGATGTC 1000
   AGTGATCACT TTCCAGTTGA GTTTAAGCTA CAGTCTTCAA GGGCCTTCAC 1050
   CAACAACAGA AAATCTGTTT CTCTCAAAAA GAGAAAAAAA GGCAATCGCT 1100
   CCTAGGTATC ACGCTCCGGA ATTC 1124

## Description

### Field of the Invention

The present invention relates to newly identified human deoxyribonuclease (DNase) protein, nucleic acid encoding such protein, the use of such protein and nucleic acid, as well as the production of such protein and nucleic acid, for example, by recombinant DNA methods.

### Backaround of the Invention

Deoxyribonuclease (DNase) is a phosphodiesterase capable of hydrolyzing polydeoxyribonucleic acid, and is known to occur in several molecular forms. Based on their biochemical properties and enzymatic activities, DNase proteins have been classified as two types, DNase I and DNase II. DNase I proteins have a pH optimum near neutrality, an obligatory requirement for divalent cations, and produce 5'-phosphate nucleotides on hydrolysis of DNA. DNase II proteins exhibit an acid pH optimum, can be activated by divalent cations, and produce 3'-phosphate nucleotides on hydrolysis of DNA.

DNase from various species have been purified to a varying degree. For example, various forms of bovine DNase I have been purified and completely sequenced (Liao, et al., J. Biol. Chem. 248:1489-1495 (1973); Oefner, et al., J. Mol. Biol. 192:605-632 (1986); Lahm. et al., J. Mol. Biol. 221:645-667 (1991)), and DNA encoding bovine DNase I has been cloned and expressed (Worrall, et al., J. Biol. Chem 265:21889-21895 (1990)). Porcine and orcine DNase I proteins also have been purified and completely sequenced (Paudel, et al., J. Biol. Chem. 261:16006-16011 (1986); Paudel, et al., J. Biol. Chem. 261:16012-16017 (1986)).

DNA encoding a human DNase 1 has been isolated and sequenced and the DNA has been expressed in recombinant host cells, thereby enabling the production of human DNase I in commercially useful quantities. Shak, et al., Proc. Natl. Acad. Sci. 87:9188-9192 (1990). The term "human DNase I" will be used hereafter to refer to the mature polypeptide disclosed in Shak, et al.

DNA encoding other polypeptides having homology to human DNase I also have been identified. Rosen, et al., PCT Patent Publication No. WO 95/30428, published November 16, 1995; Parrish, et al., Hum. Mol. Genet. 4:1557-1564 (1995).

DNase I has a number of known utilities and has been used for therapeutic purposes. Its principal therapeutic use has been to reduce the viscoelasticity of pulmonary secretions (mucus) in such diseases as pneumonia and cystic fibrosis (CF), thereby aiding in the clearing of respiratory airways. See e.g., Lourenco. et al., Arch. Intern. Med. 142:2299-2308 (1982); Shak, et al., Proc. Natl. Acad. Sci. 87:9188-9192 (1990); Hubbard, et al., New Engl. J. Med. 326:812-815 (1992); Fuchs, et al., New Engl. J. Med. 331:637-642 (1994); Bryson, et al., Drugs 48:894-906 (1994). Mucus also contributes to the morbidity of chronic bronchitis, asthmatic bronchitis, bronchiectasis, emphysema, acute and chronic sinusitis, and even the common cold.

The pulmonary secretions of persons having such diseases are complex materials, that include mucus glycoproteins, mucopolysaccharides, proteases, actin, and DNA. DNase I is effective in reducing the viscoelasticity of pulmonary secretions by hydrolyzing, or degrading, high-molecular-weight DNA that is present in such secretions. Shak, et al., Proc. Natl. Acad. Sci. 87:9188-9192 (1990); Aitken, et al., J. Am. Med. Assoc. 267:1947-1951 (1992). The DNA-hydrolytic activity of DNase I in pulmonary secretions may be reduced, however, as a result of the interaction of the DNase I with actin. Lazarides, et al., Proc. Natl. Acad. Sci. 71:4742-4746 (1974); Mannherz, et al.. Eur. J. Biochem. 104:367-379 (1980). Accordingly, forms of DNase I that bind actin with lower affinity than human native DNase I, but that still possess DNA-hydrolytic activity should be useful therapeutic agents, especially in the treatment of patients having pulmonary secretions that comprise relatively large amounts of actin. Variants of human DNase I having reduced affinity for actin have been prepared synthetically and shown to be more potent than the native enzyme in reducing the viscosity of sputum of cystic fibrosis patients. Lazarus, et al., PCT Pulication WO96/26279, published 29 August 1996.

### Summary of the Invention

The present invention provides a novel DNase, as well as analogs and variants thereof, that have DNA-hydrolytic activity. This novel polypeptide, referred to as LS-DNase, is of human origin.

The invention also provides nucleic acids encoding LS-DNase, recombinant vectors comprising such nucleic acids, recombinant host cells transformed with those nucleic acids or vectors, and processes for producing LS-DNase by means of recombinant DNA technology. The invention includes the use of such nucleic acids and vectors for in vivo or ex vivo gene therapy.

The invention also provides pharmaceutical compositions comprising LS-DNase, optionally together with a pharmaceutically acceptable excipient, as well as substantially purified antibodies that are capable of binding to LS-DNase.

The invention also provides medicaments for reducing the viscoelasticity or viscous consistency of DNA-containing material in a patient. The invention is particularly directed to a medicament for treating a patient having a disease such as cystic fibrosis, chronic bronchitis, pneumonia, bronchiectasis, emphysema, asthma, or systemic lupus erythematosus. The invention also is directed to the use of LS-DNase in in vitro diagnostic assays of a viscous material (e.g., sputum) from a patient.

These and other aspects of the invention will be apparent to the ordinary skilled artisan upon consideration of the following detailed description.

### Brief Description of the Figures

Figure 1 shows the nucleotide sequence (SEQ. ID. NO: 1) and deduced amino acid sequence (SEQ. ID. NO: 2) of LS-DNase. The predicted leader (signal) amino acid sequence is underlined and the start of the mature protein is indicated by the arrowhead.
Figure 2 shows an alignment of the amino acid sequences of human LS-DNase (SEQ. ID. NO: 3) and human DNase I (SEQ. ID. NO: 4). Identical amino acid residues are boxed, conservative amino acid substitutions are indicated by a dot (^{.}), and conserved cysteine residues are indicated by arrowheads. Two potential glycosylations sites in human DNase I are indicated by asterisks (^{.}). Amino acid residues of human DNase I that are involved in actin binding are shaded. Conserved catalytic residues are in inverted text (white on black).
Figure 3 shows the nucleotide sequence (SEQ. ID. NO: 11) of murine LS-DNase. The ATG start codon for the predicted protein is indicated by the arrowhead, and the nucleotide sequence encoding the predicted leader (signal) amino acid sequence of the protein is underlined.

### Detailed Description

The various aspects of the present invention are accomplished by first providing isolated DNA comprising the nucleotide coding sequence for LS-DNase. By providing the full nucleotide coding sequence for LS-DNase, the invention enables the production of LS-DNase by means of recombinant DNA technology, thereby making available for the first time sufficient quantities of substantially pure LS-DNase protein for diagnostic and therapeutic uses.

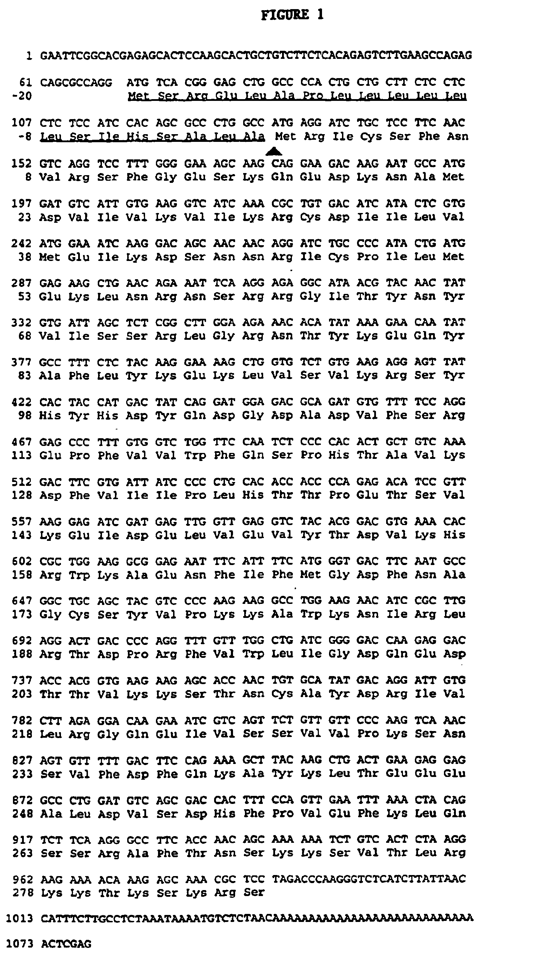

As used herein, the term "LS-DNase" refers to the polypeptide having the amino acid sequence of the mature protein set forth in Figure 1, as well as modified and variant forms thereof as described herein. The term "human LS-DNase" refers to the polypeptide having the amino acid sequence of the mature protein set forth in Figure 1.

Modified and variant forms of LS-DNase are produced in vitro by means of chemical or enzymatic treatment or in vivo by means of recombinant DNA technology. Such polypeptides differ from human LS-DNase, for example, by virtue of one or more amino acid substitutions, insertions, and/or deletions, or in the extent or pattern of glycosylation, but substantially retain a biological activity of LS-DNase. Preferably, the modified and variant forms of LS-DNase have DNA-hydrolytic activity that is substantially the same as that of human LS-DNase.

A "variant" or "amino acid sequence variant" of LS-DNase is a polypeptide that comprises an amino acid sequence different from that of human LS-DNase. Generally, a variant will possess at least 80% sequence identity (homology), preferably at least 90% sequence identity, more preferably at least 95% sequence identity, and most preferably at least 98% sequence identity with human LS-DNase. Percentage sequence identity is determined, for example, by the Fitch, et al., Proc. Natl. Acad. Sci. USA 80:1382-1386 (1983), version of the algorithm described by Needleman, et al., J. Mol. Biol. 48:443-453 (1970), after aligning the sequences to provide for maximum homology. Such variants include naturally occurring allelic forms of human LS-DNase that are of human origin as well as natuarlly occurring homologs of human LS-DNase that are found in other animal species.

"DNA-hydrolytic activity" refers to the enzymatic activity of a DNase in hydrolyzing (cleaving) substrate DNA to yield 5'-phosphorylated oligonucleotide end products. DNA-hydrolytic activity is readily determined by any of several different methods known in the art, including analytical polyacrylamide and agarose gel electrophoresis, hyperchromicity assay (Kunitz, J. Gen. Physiol. 33:349-362 (1950); Kunitz, J. Gen. Physiol. 33:363-377 (1950)), or methyl green assay (Kurnick, Arch. Biochem. 29:41-53 (1950); Sinicropi, et al., Anal. Biochem. 222:351-358 (1994)).

For convenience, substitutions, insertions, and/or deletions in the amino acid sequence of human LS-DNase are usually made by introducing mutations into the corresponding nucleotide sequence of the DNA encoding human LS-DNase, for example by site-directed mutagenesis. Expression of the mutated DNA then results in production of the variant LS-DNase, having the desired amino acid sequence.

Whereas any technique known in the art can be used to perform site-directed mutagenesis, e.g. as disclosed in Sambrook, et al., Molecular Cloning: A Laboratory Manual, Second Edition (Cold Spring Harbor Laboratory Press, New York (1989)), oligonucleotide-directed mutagenesis is the preferred method for preparing the LS-DNase variants of this invention. This method, which is well known in the art (Zoller, et al., Meth. Enzymol. 100:4668-500 (1983); Zoller, et al., Meth. Enzymol. 154:329.350 (1987); Carter. Meth. Enzymol. 154:382-403 (1987); Kunkel, et al., Meth. Enzymol. 151:367.382 (1987); Horwitz, et al., Meth. Enzymol. 185:599-611 (1990)), is particularly suitable for making substitution variants, although it may also be used to conveniently prepare deletion and insertion variants. as well as variants having multiple substitution. insertion, and/or deletion mutations.

Briefly, in carrying out site-directed mutagenesis of DNA encoding human LS-DNase (or a variant thereof), the DNA is altered by first hybridizing an oligonucleotide encoding the desired mutation to a single strand of the DNA. After hybridization, a DNA polymerase is used to synthesize an entire second strand, using the hybridized oligonucleotide as a primer, and using the single strand of the DNA as a template. Thus, the oligonucleotide encoding the desired mutation is incorporated in the resulting double-stranded DNA.

Oligonucleotides may be prepared by any suitable method, such as by purification of a naturally occurring DNA or by in vitro synthesis. For example, oligonucleotides are readily synthesized using various techniques in organic chemistry, such as described by Narang, et al., Meth. Enzymol. 68:90-98 (1979); Brown, et al., Meth. Enzymol. 68:109-151 (1979); Caruthers, et al., Meth. Enzymol. 154:287-313 (1985). The general approach to selecting a suitable oligonucleotide for use in site-directed mutagenesis is well known. Typically, the oligonucleotide will contain 10-25 or more nucleotides, and will include at least 5 nucleotides on either side of the sequence encoding the desired mutation so as to ensure that the oligonucleotide will hybridize preferentially at the desired location to the single-stranded DNA template molecule.

"Polymerase chain reaction," or "PCR," generally refers to a method for amplification of a desired nucleotide sequence in vitro, as described, for example, in U.S. Pat. No. 4,683,195. In general, the PCR method involves repeated cycles of primer extension synthesis, using oligonucleotide primers capable of hybridizing preferentially to a template nucleic acid.

PCR mutagenesis (Higuchi, in PCR Protocols, pp.177-183 (Academic Press. 1990); Vallette, et al., Nuc. Acids Res. 17:723-733 (1989)) is also suitable for making the variants of LS-DNase. Briefly, when small amounts of template DNA are used as starting material in a PCR, primers that differ slightly in sequence from the corresponding region in the template DNA can be used to generate relatively large quantities of a specific DNA fragment that differs from the template sequence only at the positions where the primers differ from the template. For introduction of a mutation into a plasmid DNA, for example, the sequence of one of the primers includes the desired mutation and is designed to hybridize to one strand of the plasmid DNA at the position of the mutation; the sequence of the other primer must be identical to a nucleotide sequence within the opposite strand of the plasmid DNA, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone. Wagner, et al., in PCR Topics, pp.69-71 (Springer-Verlag, 1991).

If the ratio of template to product amplified DNA is extremely low, the majority of product DNA fragments incorporate the desired mutation(s). This product DNA is used to replace the corresponding region in the plasmid that served as PCR template using standard recombinant DNA methods. Mutations at separate positions can be introduced simultaneously by either using a mutant second primer, or performing a second PCR with different mutant primers and ligating the two resulting PCR fragments simultaneously to the plasmid fragment in a three (or more)-part ligation.

Another method for preparing variants, cassette mutagenesis, is based on the technique described by Wells et al., Gene, 34:315-323 (1985). The starting material is the plasmid (or other vector) comprising the DNA sequence to be mutated. The codon(s) in the starting DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. The resulting plasmid contains the mutated DNA sequence.

The presence of mutation(s) in a DNA is determined by methods well known in the art, including restriction mapping and/or DNA sequencing. A preferred method for DNA sequencing is the dideoxy chain termination method of Sanger, et al., Proc. Natl. Acad. Sci. USA 12:3918-3921 (1979).

DNA encoding LS-DNase is inserted into a replicable vector for further cloning or expression. "Vectors" are plasmids and other DNAs that are capable of replicating within a host cell, and as such, are useful for performing two functions in conjunction with compatible host cells (a vector-host system). One function is to facilitate the cloning of nucleic acid that encodes LS-DNase, i.e., to produce usable quantities of the nucleic acid. The other function is to direct the expression of LS-DNase. One or both of these functions are performed by the vector in the particular host cell used for cloning or expression. The vectors will contain different components depending upon the function they are to perform.

The LS-DNase of the present invention may be in the form of a preprotein wherein the DNase includes a leader or signal sequence, or may be in the form of a mature protein which lacks a leader or signal sequence. The LS-DNase also may be in the form of a fusion protein wherein additional amino acid residues are covalently joined to the amino- or carboxy-terminus of the preprotein or mature form of the DNase.

To produce LS-DNase, an expression vector will comprise DNA encoding LS-DNase, as described above, operably linked to a promoter and a ribosome binding site. The LS-DNase then is expressed directly in recombinant cell culture, or as a fusion with a heterologous polypeptide, preferably a signal sequence or other polypeptide having a specific cleavage site at the junction between the heterologous polypeptide and the LS-DNase amino acid sequence.

"Operably linked" refers to the covalent joining of two or more DNA sequences, by means of enzymatic ligation or otherwise, in a configuration relative to one another such that the normal function of the sequences can be performed. For example, DNA for a presequence or secretory leader is operably linked to DNA for a polypeptide if it is expressed as a preprotein that participates in the secretion of the polypeptide: a promoter or enhancer is operably linked to a coding sequence if it affects the transcription of the sequence: or a ribosome binding site is operably linked to a coding sequence if it is positioned so as to facilitate translation. Generally, "operably linked" means that the DNA sequences being linked are contiguous and, in the case of a secretory leader, contiguous and in reading phase. Linking is accomplished by ligation at convenient restriction sites. If such sites do not exist, then synthetic oligonucleotide adaptors or linkers are used, in conjunction with standard recombinant DNA methods.

Prokaryotes (e.g., E. coli, strains of Bacillus, Pseudomonas, and other bacteria) are the preferred host cells for the initial cloning steps of this invention. They are particularly useful for rapid production of large amounts of DNA. for production of single-stranded DNA templates used for site-directed mutagenesis. and for DNA sequencing of the variants generated. Prokaryotic host cells also may be used for expression of DNA encoding LS-DNase. Polypeptides that are produced in prokaryotic cells typically will be non-glycosylated.

In addition, LS-DNase may be expressed in eukaryotic host cells, including eukaryotic microbes (e.g., yeast) or cells derived from an animal or other multicellular organism (e.g., Chinese hamster ovary cells, and other mammalian cells), or in live animals (e.g., cows, goats, sheep). Insect cells also may be used.

Cloning and expression methodologies are well known in the art. Examples of prokaryotic and eukaryotic host cells, and starting expression vectors, suitable for use in producing LS-DNase are, for example, those disclosed in Shak, PCT Patent Publication No. WO 90/07572, published July 12, 1990. To obtain expression of LS-DNase, an expression vector of the invention is introduced into host cells by transformation or transfection, and the resulting recombinant host cells are cultured in conventional nutrient media, modified as appropriate for inducing promoters, selecting recombinant cells, or amplifying LS-DNase DNA. The culture conditions, such as temperature, pH, and the like, are those previously used with the host cell, and as such will be apparent to the ordinarily skilled artisan.

"Transformation" and "transfection" are used interchangeably to refer to the process of introducing DNA into a cell. Following transformation or transfection, the DNA may integrate into the host cell genome, or may exist as an extrachromosomal element. If prokaryotic cells or cells that contain substantial cell wall constructions are used as hosts, the preferred methods of transfection of the cells with DNA is the calcium treatment method described by Cohen et al., Proc. Natl. Acad. Sci. 69:2110-2114 (1972) or the polyethylene glycol method of Chung et al., Nuc. Acids. Res. 16:3580 (1988). If yeast are used as the host, transfection is generally accomplished using polyethylene glycol, as taught by Hinnen, Proc. Natl. Acad. Sci. U.S.A., 75; 1929-1933 (1978). If mammalian cells are used as host cells, transfection generally is carried out by the calcium phosphate precipitation method, Graham, et al., Virology 52:546 (1978), Gorman, et al., DNA and Protein Eng. Tech. 2:3-10 (1990). However, other known methods for introducing DNA into prokaryotic and eukaryotic cells, such as nuclear injection, electroporation, or protoplast fusion also are suitable for use in this invention.

Particularly useful in this invention are expression vectors that provide for the transient expression in mammalian cells of DNA encoding LS-DNase. In general, transient expression involves the use of an expression vector that is able to efficiently replicate in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties. Wong, et al., Science 228:810-815 (1985); Lee, et al., Proc. Nat Acad. Sci. USA 82:4360-4364 (1985); Yang, et al., Cell 47:3-10 (1986). Thus, transient expression systems are conveniently used for expressing the DNA encoding amino acid sequence variants of LS-DNase, in conjunction with assays to identify those variants that have such useful properties as increased half-life or decreased immunogenicity in vivo, increased DNA hydrolytic activity, or increased resistance to inhibition by actin. The inhibition of DNase activity by actin is readily determined using assays and methods known in the art and as described herein.

LS-DNase preferably is secreted from the host cell in which it is expressed, in which case the variant is recovered from the culture medium in which the host cells are grown. In that case, it may be desirable to grow the cells in a serum free culture medium, since the absence of serum proteins and other serum components in the medium may facilitate purification of the variant. If it is not secreted, then the LS-DNase is recovered from lysates of the host cells. When the LS-DNase is expressed in a host cell other than one of human origin, the variant will be completely free of proteins of human origin. In any event, it will be necessary to purify the LS-DNase from recombinant cell proteins in order to obtain substantially homogeneous preparations of the LS-DNase. For therapeutic uses, the purified LS-DNase preferably will be greater than 99% pure (i.e., any other proteins will comprise less than 1% of the total protein in the purified composition).

It is further contemplated that LS-DNase may be produced by a method involving homologous recombination and amplification, for example, as described in PCT Patent Publication No. WO 91/06667, published May 16, 1991. Briefly, this method involves transforming cells containing an endogenous gene encoding LS-DNase with a homologous DNA, which homologous DNA comprises (1) an amplifiable gene (e.g., a gene encoding dihydrofolate reductase (DHFR)), and (2) at least one flanking sequence, having a length of at least about 150 base pairs, which is homologous with a nucleotide sequence in the cell genome that is within or in proximity to the gene encoding LS-DNase. The transformation is carried out under conditions such that the homologous DNA integrates into the cell genome by recombination. Cells having integrated the homologous DNA then are subjected to conditions which select for amplification of the amplifiable gene, whereby the LS-DNase gene amplified concomitantly. The resulting cells then are screened for production of desired amounts of LS-DNase. Flanking sequences that are in proximity to a gene encoding LS-DNase are readily identified, for example, by the method of genomic walking, using as a starting point the nucleotide sequence of LS-DNase shown in Figure 1. Spoerel, et al., Meth. Enzymol. 152:598-603 (1987).

Generally, purification of LS-DNase is accomplished by taking advantage of the differential physicochemical properties of the LS-DNase as compared to the contaminants with which it may be associated. For example, as a first step, the culture medium or host cell lysate is centrifuged to remove particulate cell debris. The LS-DNase thereafter is purified from contaminant soluble proteins and polypeptides, for example, by ammonium sulfate or ethanol precipitation, gel filtration (molecular exclusion chromatography), ionexchange chromatography, hydrophobic chromatography, immunoaffinity chromatography (e.g., using a column comprising anti-LS-DNase antibodies coupled to Sepharose), tentacle cation exchange chromatography (Frenz, et al., U.S. Patent No. 5,279,823, issued January 18, 1994), reverse phase HPLC, and/or gel electrophoresis.

In some host cells (especially bacterial host cells) the LS-DNase may be expressed initially in an insoluble, aggregated form (referred to in the art as "refractile bodies" or "inclusion bodies") in which case it will be necessary to solubilize and renature the LS-DNase in the course of its purification. Methods for solubilizing and renaturing recombinant protein refractile bodies are known in the art (see e.g., Builder, et al., U.S. Patent No. 4,511,502, issued April 16, 1985).

In another embodiment of this invention, covalent modifications are made directly to LS-DNase to give it a desired property (for example, increased half-life or decreased immunogenicity in vivo, increased DNA hydrolytic activity, or increased resistance to inhibition by actin), and may be made instead of or in addition to the amino acid sequence substitution, insertion, and deletion mutations described above.

Covalent modifications are introduced by reacting targeted amino acid residues of LS-DNase with an organic derivatizing agent that is capable of reacting with selected amino acid side-chains or N- or C-terminal residues. Suitable derivatizing agents and methods are well known in the art. Covalent coupling of glycosides to amino acid residues of the protein may be used to modify or increase the number or profile of carbohydrate substituents.

The covalent attachment of agents such as polyethylene glycol (PEG) or human serum albumin to LS-DNase may reduce immunogenicity and/or toxicity of the LS-DNase and/or prolong its half-life, as has been observed with other proteins. Abuchowski, et al., J. Biol. Chem. 252:3582-3586 (1977); Poznansky, et al., FEBS Letters 239:18-22 (1988); Goodson, et al., Biotechnology 8:343-346 (1990); Katre, J. Immunol, 144:209-213 (1990); Harris, Polyethylene Glycol Chemistry (Plenum Press, 1992). In addition, modification of LS-DNase by these agents at or adjacent to (i.e., within about five amino acid residues of) an amino acid residue that affects actin binding may produce a variant having increased resistance to inhibition by actin. As another example, the variant or modified form of LS-DNase may comprise an amino acid sequence mutation or other covalent modification that reduces the susceptibility of the variant to degradation by proteases (e.g., neutrophil elastase) that may be present in sputum and other biological materials, as compared to human LS-DNase.

Antibodies to LS-DNase are produced by immunizing an animal with LS-DNase or a fragment thereof, optionally in conjunction with an immunogenic polypeptide, and thereafter recovering antibodies from the serum of the immunized animals. Alternatively, monoclonal antibodies are prepared from cells of the immunized animal in conventional fashion. The antibodies also can be made in the form of chimeric (e.g., humanized) or single chain antibodies or Fab fragments, using methods well known in the art. Preferably, the antibodies will bind to LS-DNase but will not substantially bind to (i.e., cross react with) other DNase proteins (such as human and bovine DNase 1). The antibodies can be used in methods relating to the localization and activity of LS-DNase, for example, for detecting LS-DNase and measuring its levels in tissues or clinical samples. Immobilized anti-LS-DNase antibodies are particularly useful in the detection of LS-DNase in clinical samples for diagnostic purposes, and in the purification of LS-DNase.

Purified LS-DNase is used to reduce the viscoelasticity of DNA-containing material, such as sputum, mucus, or other pulmonary secretions. LS-DNase is particularly useful for the treatment of patients with pulmonary disease who have abnormal viscous or inspissated secretions and conditions such as acute or chronic bronchial pulmonary disease, including infectious pneumonia, bronchitis or tracheobronchitis, bronchiectasis, cystic fibrosis, asthma, tuberculosis, and fungal infections. For such therapies, a solution or finely divided dry preparation of the LS-DNase is instilled in conventional fashion into the airways (e.g., bronchi) or lungs of a patient, for example by aerosolization.

LS-DNase also is useful for adjunctive treatment of abscesses or severe closed-space infections in conditions such as empyema, meningitis, abscess, peritonitis, sinusitis, otitis, periodontitis, pericarditis, pancreatitis, cholelithiasis, endocarditis and septic arthritis, as well as in topical treatments in a variety of inflammatory and infected lesions such as infected lesions of the skin and/or mucosal membranes, surgical wounds, ulcerative lesions and bums. LS-DNase may improve the efficacy of antibiotics used in the treatment of such infections (e.g., gentamicin activity is markedly reduced by reversible binding to intact DNA).

LS-DNase also is useful for preventing the new development and/or exacerbation of respiratory infections, such as may occur in patients having cystic fibrosis, chronic bronchitis, asthma, pneumonia, or other pulmonary disease, or patients whose breathing is assisted by ventilator or other mechanical device, or other patients at risk of developing respiratory infections, for example post-surgical patients.

LS-DNase also is useful for the treatment for systemic lupus erythematosus (SLE), a life-threatening autoimmune disease characterized by the production of diverse autoantibodies. DNA is a major antigenic component of the immune complexes. In this instance, the LS-DNase may be given systemically, for example by intravenous, subcutaneous, intrathecal, or intramuscular administration to the affected patient.

Finally, LS-DNase is useful for the treatment of other non-infected conditions in which there is an accumulation of cellular debris that includes cellular DNA, such as pyelonephritis and tubulo-interstitial kidney disease.

LS-DNase can be formulated according to known methods to prepare therapeutically useful compositions. Typically, the LS-DNase is formulated with a physiologically acceptable excipient (or carrier) for therapeutic use. Such excipients are used, for example, to provide liquid formulations and sustained-release formulations of LS-DNase. A preferred therapeutic composition is a solution of LS-DNase in a buffered or unbuffered aqueous solution, and preferably is an isotonic salt solution such as 150 mM sodium chloride containing 1.0 mM calcium chloride at pH 7. These solutions are particularly adaptable for use in commercially-available nebulizers including jet nebulizers and ultrasonic nebulizers useful for administration directly into the airways or lungs of an affected patient. Another preferred therapeutic composition is a dry powder of LS-DNase, preferably prepared by spray-drying of a solution of the LS-DNase, essentially as described in PCT Publication WO95/23613. In all cases, it is desirable that the therapeutic compositions be sterile. Preferably, the therapeutic compositions are disposed in a container fabricated of plastic or other non-glass material.

In a further embodiment, the therapeutic composition comprises cells actively producing LS-DNase. Such cells may be directly introduced into the tissue of a patient, or may be encapsulated within porous membranes which are then implanted in a patient (see e.g., Aebischer, et al., U.S. Patent No. 4,892,538, issued January 9, 1990; Aebischer, et al., U.S. Patent No. 5,283,187, issued February 1, 1994), in either case providing for the delivery of the LS-DNase into areas within the body of the patient in need of increased concentrations of DNA-hydrolytic activity. For example, the patient's own cells could be transformed, either in vivo or ex vivo, with DNA encoding LS-DNase, and then used to produce the LS-DNase directly within the patient. This latter method is commonly referred to as gene therapy.

The therapeutically effective amount of LS-DNase will depend, for example, upon the amount of DNA and actin in the material to be treated, the therapeutic objectives, the route of administration, and the condition of the patient. Accordingly, it will be necessary for the therapist to titer the dosage and modify the route of administration as required to obtain the optimal therapeutic effect. In view of its reduced inhibition by actin and consequential increased DNA-hydrolytic activity in the presence of actin relative to human DNase 1, the amount of LS-DNase required to achieve a therapeutic effect may be less than the amount of human DNase I necessary to achieve the same effect under the same conditions. Generally, the therapeutically effective amount of LS-DNase will be a dosage of from about 0.1 µg to about 5 mg of the variant per kilogram of body weight of the patient, administered within pharmaceutical compositions, as described herein.

LS-DNase optionally is combined with or administered in concert with one or more other pharmacologic agents used to treat the conditions listed above, such as antibiotics, bronchodilators, antiinflammatory agents, mucolytics (e.g. n-acetyl-cysteine), actin binding or actin severing proteins (e.g., gelsolin; Matsudaira et al., Cell 54:139.140 (1988); Stossel. et al., PCT Patent Publication No. WO 94/22465. published October 13, 1994; protease inhibitors; or gene therapy product (e.g., comprising the cystic fibrosis transmembrane conductance regulator (CFTR) gene); Riordan, et al., Science 245:1066-1073 (1989)).

This invention also provides methods for determining the presence of a nucleic acid molecule encoding LS-DNase in test samples prepared from cells, tissues, or biological fluids, comprising contacting the test sample with isolated DNA comprising all or a portion of the nucleotide coding sequence for LS-DNase and determining whether the isolated DNA hybridizes to a nucleic acid molecule in the test sample. DNA comprising all or a portion of the nucleotide coding sequence for LS-DNase is also used in hybridization assays to identify and to isolate nucleic acids sharing substantial sequence identity to the coding sequence for LS-DNase, such as nucleic acids that encode naturally-occurring allelic variants of LS-DNase.

Also provided is a method for amplifying a nucleic acid molecule encoding LS-DNase that is present in a test sample, comprising the use of an oligonucleotide having a portion of the nucleotide coding sequence for LS-DNase as a primer in a polymerase chain reaction.

The following examples are offered by way of illustration only and are not intended to limit the invention in any manner.

### EXAMPLE 1

### Cloning LS-DNase cDNA

Full-length cDNA encoding LS-DNase was identified by screening a human liver cDNA library (in λ-UniZAP XR, Stratagene, La Jolla, CA) with a mixture of the following oligonucleotide probes, each of which had been end-labeled with T4 polynucleotide kinase and γ-³²P-adenosine triphosphate to a high specific radioactivity:
5'-ACTGTAGTTTAAATTCAACTGGAAAGTGGTCGCTGACATCCAGGG-3' (SEQ. ID. NO: 5)
5'-GATGTCATTGTGAAGGTCATCAAACGCTGTGACATCATACTCGTG-3' (SEQ. ID. NO: 6)
5'-GTGTTTTCCAGGGGAGCCCTTTGTGGTCTGGTTCCAATCTCCCCA-3' (SEQ. ID. NO: 7)
S'-CTGGAGGTCTCCCAGCACTGGCAGAGCAAGGACGTGATCCTGCTT-3' (SEQ. ID. NO: 8)
5'-GCCCAGCATCATCGCGAAGTTCCTGGCTGGCTATCACCTCGCGCT-3' (SEQ. ID. NO: 9)
S'-CCAGTACAAGGAGATGTACCTCTTCGTTTACAGGAAAGACGCCGT.3'(SEQ. ID.NO: 10)
The first three of the oligonucleotide probes listed above (SEQ. ID. NOS: 5-7) comprise portions of the EST sequences having accession codes T68985, T69063, HSAAACIFW, T73558, T61400, T73653, and T61368 in the Genbank database. The other two oligonucleotide probes listed above (SEQ. ID. NOS: 9-10) comprise portions of the EST sequences having accession codes R78020 and H42990 in the Genbank database.

Hybridization of the probes to the cDNA library was carried out under low stringency conditions (in 20% vol/vol formamide, 5X SSPE, 5X Denhardt's solution, 0.1% sodium dodecyl sulfate (SDS), and 100µg/ml sonicated salmon sperm DNA), at 42°C. Post hybridization washes were carried out in 2X SSC, 0.1% SDS, at 42°C. 1X SSPE is 150mM NaCl, 10mM sodium phosphate. 1mM EDTA, pH 7.4. IX Denhardt's solution is 0.02% Ficoll, 0.02% bovine serum albumin, and 0.02% polyvinyl-pyrrolidone. 1X SSC is 0.15 M NaCl, 0.015 M sodium citrate, pH 7.0.

Hybridization-positive clones were found only with the first three of the oligonucleotide probes listed above (SEQ. ID. NOS: 5-7). Those clones were convened into phagemid-based sequences following standard procedures (Stratagene, La Jolla, California, USA) and were sequenced. The largest inserted nucleotide sequence found amongst the hybridization-positive clones was 1079 base-pairs in length, including an open reading frame of 915 base-pairs that encodes a predicted protein that is 305 amino acid residues in length. The nucleotide sequence of the 1079 base-pair insert (SEQ. ID. NO: 1) and the amino acid sequence of predicted protein (SEQ. ID. NO: 2) are shown in Figure 1.

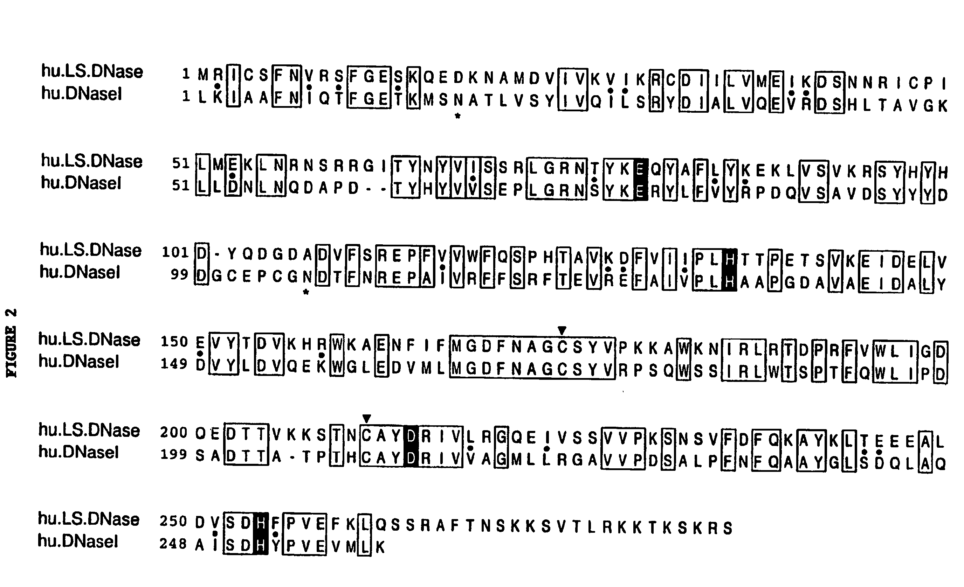

The predicted protein includes a signal sequence that is 20 amino acid residues in length. Cleavage of the signal sequence releases the mature protein (LS-DNase), which has a predicted molecular weight of 33,400 Daltons and a predicted pI of 9.7. The amino acid sequence of LS-DNase is 46% identical to the amino acid sequence of human DNase I (Figure 2).

LS-DNase contains five cysteine residues, two of which (Cys-174 and Cys-211) coincide with a pair of cysteine residues in human DNase I that are disulfide bonded, suggesting that LS-DNase and human DNase I may have similar tertiary structures. Amino acid residues known to be important for the DNA-hydrolytic activity of human DNase I are conserved in LS-DNase, including the active site histidine residues His-135 and His-254. Conversely, several amino acid residues known to comprise the actin-binding site of human DNase 1 are not conserved in LS-DNase. In particular, Val-67 and Ala-114 of human DNase 1 are replaced by Ile-69 and Phe-115, respectively, at the homologous positions in LS-DNase. An analogous replacement of Val-67 by Ile occurs in rat DNase I, which has approximately 1000-fold lower affinity for actin as compared to human DNase I.

### EXAMPLE 2

### Expression of LS-DNase cDNA

The cDNA encoding LS-DNase was subcloned into a mammalian expression vector pRK5 (Gorman, et al., DNA and Protein Engineering Techniques 2:1 (1990); European Patent Publication EP 307,247, published March 15, 1989). The resulting recombinant vector is referred to as pRK5/LS-DNase. Human embryonic kidney 293 cells (American Type Culture Collection, CRL 1573) were grown in serum-containing Dulbecco Modified Eagle's medium (DMEM) to 70% confluency and then transiently transfected with pRK5/LS-DNase, or as a control, pRK5 alone. 24 hours post-transfection, the cells were washed with phosphate buffered saline and transferred to serum-free medium containing insulin. 72-96 hours later, conditioned medium was collected from the cell cultures and concentrated approximately 10-fold. Proteins expressed in the cell cultures were analyzed by SDS-polyacrylamide gel electrophoresis (SDS-PAGE).

Cells trartsfected with pRK-5/LS-DNase were found to produce a unique, sharply resolving protein of about 32,000 - 34,000 Daltons, that was not produced in cells transfected with pRK5 alone. The molecular weight size of this protein is in good agreement with that predicted for LS-DNase.

### EXAMPLE 3

### Biological Activity of LS-DNase

Concentrated cell culture supernatants, prepared as described above, were tested for DNase activity in a hyperchromicity assay (Kunitz, J. Gen. Physiol. 33:349-362 (1950); Kunitz. J. Gen. Physiol. 33:363-377 (1950)), and a methyl green assay (Kurnick, Arch. Biochem. 29:41-53 (1950); Sinicropi, et al., Anal. Biochem. 222:351-358 (1994)). In both assays, DNase activity was detected in the supernatants from cells transfected with pRK5/LS-DNase, but not in the supernatants from cells transfected with pRK5 alone.

### EXAMPLE 4

### Resistance to Actin Inhibition

To determine whether the DNA-hydrolytic activity of LS-DNase is inhibited by actin, a plasmid nicking assay was used. This assay measures the conversion of supercoiled double-stranded pBR322 plasmid DNA to nicked, linear, and degraded forms. Specifically, various DNase samples were added to 20 µl of solution containing 25 µg/ml supercoiled double-stranded pBR322 DNA in 25mM HEPES buffer, 1mM MgCl₂, 1mM CaCl₂, 100 µg/ml bovine serum albumin, and the samples were incubated for 10 minutes at 21 °C. To determine inhibition by actin, the DNase samples were pre-incubated with actin for 15 minutes at 21°C. prior to being added to the solution of pBR322 DNA. Reactions were stopped by the addition of EDTA to a final concentration of 10mM, together with xylene cyanol, bromphenol blue, and glycerol. The integrity of the pBR322 DNA was analyzed by electrophoresis of the reaction mixtures on 0.8% weight/vol. agarose gels. After electrophoresis, the gels were stained with a solution of ethidium bromide and the DNA in the gels was visualized by ultraviolet light.

As expected, human DNase I converted the starting plasmid DNA to degraded forms, and the DNA-hydrolytic activity of human DNase I was inhibited by added actin in a concentration-dependent manner. LS-DNase converted the starting plasmid DNA to nicked, linear, and degraded forms, but the DNA-hydrolytic activity of LS-DNase was not inhibited by concentrations of actin that fully inhibited human DNase I.

### EXAMPLE 5

### Pattern of Expression of LS-DNase in Human Tissue

Northern blots of various human tissues were performed using a radiolabeled probe comprising a portion of the coding sequence of the cloned LS-DNase cDNA. Expression of LS-DNase messenger RNA (mRNA) was found to be highest in liver and spleen. LS-DNase mRNA either was poorly expressed or not expressed in other tissues examined. No LS-DNase mRNA was detectable in pancreas tissue.

Northern blots of various human tissues also were performed using a radiolabeled probe comprising a portion of the nucleotide coding sequence for human DNase I. In contrast to LS-DNase mRNA. human DNase I mRNA appeared to be exclusively expressed in pancreas tissue.

### EXAMPLE 6

### Cloning of LS-DNase Variant

A 649 base-pair EcoRI-PstI fragment of the coding sequence of the cloned LS-DNase cDNA was used to screen a murine liver cDNA library (in λ-gt10. Clontech, Palo Alto, California. USA). From about two million clones screened, more than 60 hybridization positive clones were identified. Partial sequencing of six random positive clones showed that they all originated from the same gene. The inserted nucleotide sequence of one of those positive clones was completely sequenced. The insert was 1124 base-pairs in length, including an open reading frame of 930 base-pairs that encodes a predicted protein, referred to as murine LS-DNase, that is 310 amino acid residues in length.

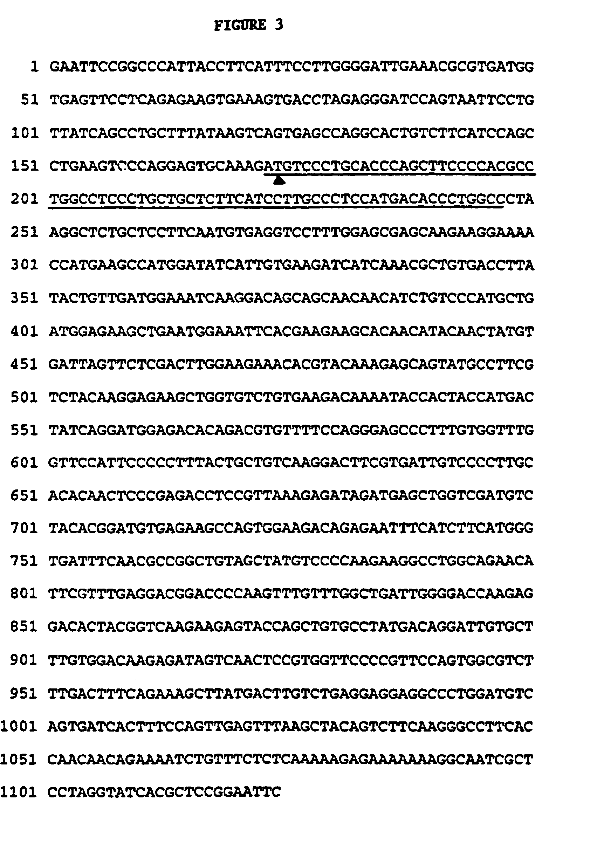

The nucleotide sequence of the 1124 base-pair insert (SEQ. ID. NO: 11) is shown in Figure 3. The open reading frame begins with the ATG codon at nucleotide 173 and continues to the stop codon at nucleotide 1103. The first 75 nucleotides of the open reading frame (the first 25 amino acid residues of the predicted protein) encode a putative signal sequence. Accordingly, the predicted murine mature LS-DNase protein is 285 amino acid residues in length, has a molecular weight of 33,100 Daltons and a predicted pl of 9.4. The amino acid sequence of the murine mature LS-DNase is 84% identical to the amino acid sequence shown in Figure 1 for human mature LS-DNase.

Northern blots of various mouse tissues were performed using a radiolabeled probe comprising a portion of the nucleotide coding sequence for murine LS-DNase. Expression of murine LS-DNase messenger RNA (mRNA) was found to be highest in liver and spleen. LS-DNase mRNA either was poorly expressed or not expressed in other tissues examined.

## Claims

1. An isolated nucleic acid molecule comprising a nucleotide sequence that encodes an amino acid sequence which has at least 80% identity with the amino acid sequence shown in Figure 1 for mature LS-deoxyribonuclease (LS-DNase) and which has DNA-hydrolytic activity.

2. The isolated nucleic acid molecule of Claim 1 comprising a nucleotide sequence that encodes an amino acid sequence which has at least 90% identity with the amino acid sequence shown in Figure 1 for mature LS-DNase and which has DNA-hydrolytic activity.

3. The isolated nucleic acid molecule of Claim 2 comprising a nucleotide sequence that encodes an amino acid sequence which has at least 95% identity with the amino acid sequence shown in Figure 1 for mature LS-DNase and which has DNA-hydrolytic activity.

4. The isolated nucleic acid molecule of Claim 3 comprising a nucleotide sequence that encodes an amino acid sequence which has at least 98% identity with the amino acid sequence shown in Figure 1 for mature LS-DNase and which has DNA-hydrolytic activity.

5. The isolated nucleic acid molecule of Claim 4 comprising a nucleotide sequence that encodes the amino acid sequence shown in Figure 1 for mature LS-DNase and which has DNA-hydrolytic activity.

6. An isolated nucleic acid molecule comprising a nucleotide sequence that encodes an amino acid sequence which differs from the amino acid sequence shown in Figure 1 for mature LS-DNase by the substitution of one amino acid for another at only a single position within the Figure 1 sequence and which has DNA-hydrolytic activity.

7. An isolated nucleic acid molecule comprising a nucleotide sequence that encodes an amino acid sequence which differs from the amino acid sequence shown in Figure 1 for mature LS-DNase by the substitution of one amino acid for another at only two positions within the Figure 1 sequence and which has DNA-hydrolytic activity.

8. An expression vector comprising a nucleotide sequence encoding the amino acid sequence shown in Figure 1 for mature LS-DNase operably linked to a promoter recognized by a host cell transformed with the vector.

9. A host cell transformed with an expression vector comprising a nucleotide sequence encoding the amino acid sequence shown in Figure 1 for mature LS-DNase.

10. A process which comprises transforming a host cell with a nucleic acid molecule that encodes a polypeptide comprising the amino acid sequence shown in Figure 1 for mature LS-DNase and culturing the host cell under conditions such that the polypeptide is produced in the host cell.

11. A method of producing LS-DNase having the amino acid sequence shown in Figure 1, which comprises:
(a) transforming a cell containing an endogenous LS-DNase gene with a homologous DNA comprising an amplifiable gene and flanking sequence of at least about 150 base pairs that is homologous with a DNA sequence within or in proximity to the endogenous LS-DNase gene, whereby the homologous DNA integrates into the cell genome by recombination;
(b) culturing the cells under conditions that select for amplification of the amplifiable gene, whereby the LS-DNase gene is also amplified; and thereafter
(c) recovering LS-DNase from the cells.

12. An isolated polypeptide comprising an amino acid sequence which has at least 80% identity with the amino acid sequence shown in Figure 1 for mature LS-DNase and which has DNA-hydrolytic activity.

13. The isolated polypeptide of Claim 12 comprising an amino acid sequence which has at least 90% identity with the amino acid sequence shown in Figure 1 for mature LS-DNase and which.has DNA-hydrolytic activity.

14. The isolated polypeptide of Claim 13 comprising an amino acid sequence which has at least 95% identity with the amino acid sequence shown in Figure 1 for mature LS-DNase and which has DNA-hydrolytic activity.

15. The isolated polypeptide of Claim 14 comprising an amino acid sequence which has at least 98% identity with the amino acid sequence shown in Figure 1 for mature LS-DNase and which has DNA-hydrolytic activity.

16. The isolated polypeptide of Claim 15 comprising the amino acid sequence shown in Figure 1 for mature LS-DNase and which has DNA-hydrolytic activity.

17. An isolated polypeptide comprising an amino acid sequence that differs from the amino acid sequence shown in Figure 1 for mature LS-DNase by the substitution of one amino acid for another at only a single position within the Figure 1 sequence, which polypeptide has DNA-hydrolytic activity.

18. The isolated polypeptide of claim 17 wherein the amino acid substitution creates a glycosylation site within the polypeptide that is not present in human LS-DNase.

19. An isolated polypeptide comprising an amino acid sequence that differs from the amino acid sequence shown in Figure 1 for mature LS-DNase by the substitution of one amino acid for another at only two positions within the Figure 1 sequence, which polypeptide has DNA-hydrolytic activity.

20. A pharmaceutical composition comprising a polypeptide according to any one of claims 12 to 19 and a physiologically acceptable excipient.

21. An antibody which binds to the amino acid sequence shown in Figure 1 for mature LS-DNase and which does not cross-react with other DNase proteins.

22. An antibody of claim 21 which is a monoclonal antibody.

23. The use of a polypeptide according to any one of claims 12 to 19 in the manufacture of a medicament for treating a pulmonary disease or disorder.

24. The use of claim 23 wherein the disease or disorder is cystic fibrosis.

25. The use of a polypeptide according to any one of claims 12 to 19 in the manufacture of a medicament for treating systemic lupus erythematosus.

## Patentansprüche

1. Isoliertes Nucleinsäuremolekül, umfassend eine Nucleotidsequenz, die für eine Aminosäuresequenz kodiert, die eine mindestens 80 %ige Identität mit der in Fig. 1 dargestellten Aminosäuresequenz für reife LS-Desoxyribonuclease (LS-DNase) aufweist und die DNA-hydrolytische Aktivität besitzt.

2. Isoliertes Nucleinsäuremolekül nach Anspruch 1, umfassend eine Nucleotidsequenz, die für eine Aminosäuresequenz kodiert, die eine mindestens 90 %ige Identität mit der in Fig. 1 dargestellten Aminosäuresequenz für reife LS-DNase aufweist und die DNA-hydrolytische Aktivität besitzt.

3. Isoliertes Nucleinsäuremolekül nach Anspruch 2, umfassend eine Nucleotidsequenz, die für eine Aminosäuresequenz kodiert, die eine mindestens 95 %ige Identität mit der in Fig. 1 dargestellten Aminosäuresequenz für reife LS-DNase aufweist und die DNA-hydrolytische Aktivität besitzt.

4. Isoliertes Nucleinsäuremolekül nach Anspruch 3, umfassend eine Nucleotidsequenz, die für eine Aminosäuresequenz kodiert, die eine mindestens 98 %ige Identität mit der in Fig. 1 dargestellten Aminosäuresequenz für reife LS-DNase aufweist und die DNA-hydrolytische Aktivität besitzt.

5. Isoliertes Nucleinsäuremolekül nach Anspruch 4, umfassend eine Nucleotidsequenz, die für die in Fig. 1 dargestellte Aminosäuresequenz für reife LS-DNase kodiert und die DNA-hydrolytische Aktivität besitzt.

6. Isoliertes Nucleinsäuremolekül , umfassend eine Nucleotidsequenz, die für eine Aminosäuresequenz kodiert, die sich von der in Fig. 1 dargestellten Aminosäuresequenz für reife LS-DNase durch die Substitution einer Aminosäure anstelle einer anderen in nur einer einzigen Position innerhalb der Sequenz von Fig. 1 unterscheidet und die DNA-hydrolytische Aktivität besitzt.

7. Isoliertes Nucleinsäuremolekül , umfassend eine Nucleotidsequenz, die für eine Aminosäuresequenz kodiert, die sich von der in Fig. 1 dargestellten Aminosäuresequenz für reife LS-DNase durch die Substitution einer Aminosäure anstelle einer anderen in nur zwei Positionen innerhalb der Sequenz von Fig. 1 unterscheidet und die DNA-hydrolytische Aktivität besitzt.

8. Expressionsvektor, umfassend eine Nucleotidsequenz, die für die in Fig. 1 dargestellte Aminosäuresequenz für reife LS-DNase kodiert, in funktioneller Verknüpfung mit einem Promotor, der von einer Wirtszelle, die mit dem Vektor transformiert ist, erkannt wird.

9. Wirtszelle, die mit einem Expressionsvektor transformiert ist, der eine Nucleotidsequenz umfasst, die für die in Fig. 1 dargestellte Aminosäuresequenz für reife LS-DNase kodiert.

10. Verfahren, umfassend das Transformieren einer Wirtszelle mit einem Nucleinsäuremolekül, das für ein Polypeptid kodiert, das die in Fig. 1 für reife LS-DNase dargestellte Aminosäuresequenz umfasst, und das Züchten der Wirtszelle unter solchen Bedingungen, dass das Polypeptid in der Wirtszelle erzeugt wird.

11. Verfahren zum Erzeugen von LS-DNase mit der in Fig. 1 dargestellten Aminosäuresequenz, umfassend
(a) das Transformieren einer Zelle, die ein endogenes LS-DNase-Gen enthält, mit einer homologen DNA, die ein amplifizierbares Gen und eine Flankierungssequenz mit mindestens etwa 150 Basenpaaren, die zu einer DNA-Sequenz innerhalb oder in der Nähe des endogenen LS-DNase-Gens homolog ist, umfasst, wobei die homologe DNA sich durch Rekombination in das Zellgenom integriert;
(b) das Züchten der Zellen unter solchen Bedingungen, dass eine Selektion in Bezug auf die Amplifikation des amplifizierbaren Gens erfolgt, wobei das LS-DNase-Gen ebenfalls amplifiziert wird; und anschließend
(c) das Gewinnen der LS-DNase aus den Zellen.

12. Isoliertes Polypeptid, umfassend eine Aminosäuresequenz, die eine mindestens 80 %ige Identität mit der in Fig. 1 dargestellten Aminosäuresequenz für reife LS-DNase aufweist und die DNA-hydrolytische Aktivität besitzt.

13. Isoliertes Polypeptid nach Anspruch 12, umfassend eine Aminosäuresequenz, die eine mindestens 90 %ige Identität mit der in Fig. 1 dargestellten Aminosäuresequenz für reife LS-DNase aufweist und die DNA-hydrolytische Aktivität besitzt.

14. Isoliertes Polypeptid nach Anspruch 13, umfassend eine Aminosäuresequenz, die eine mindestens 95 %ige Identität mit der in Fig. 1 dargestellten Aminosäuresequenz für reife LS-DNase aufweist und die DNA-hydrolytische Aktivität besitzt.

15. Isoliertes Polypeptid nach Anspruch 14, umfassend eine Aminosäuresequenz, die eine mindestens 98 %ige Identität mit der in Fig. 1 dargestellten Aminosäuresequenz für reife LS-DNase aufweist und die DNA-hydrolytische Aktivität besitzt.

16. Isoliertes Polypeptid nach Anspruch 15, das die in Fig. 1 dargestellte Aminosäuresequenz für reife LS-DNase aufweist und das DNA-hydrolytische Aktivität besitzt.

17. Isoliertes Polypeptid, umfassend eine Aminosäuresequenz, die sich von der in Fig. 1 dargestellten Aminosäuresequenz für reife LS-DNase durch die Substitution einer Aminosäure anstelle einer anderen in nur einer einzigen Position innerhalb der Sequenz von Fig. 1 unterscheidet, wobei das Polypeptid DNA-hydrolytische Aktivität besitzt.

18. Isoliertes Polypeptid nach Anspruch 17, wobei die Aminosäuresubstitution eine Glycosylierungsstelle innerhalb des Polypeptids schaft, die in humaner LS-DNase nicht vorhanden ist.

19. Isoliertes Polypeptid, umfassend eine Aminosäuresequenz, die sich von der Fig. 1 dargestellten Aminosäuresequenz für reife LS-DNase durch die Substitution einer Aminosäure anstelle einer anderen in nur zwei Positionen innerhalb der Sequenz von Fig. 1 unterscheidet, wobei das Polypeptid DNA-hydrolytische Aktivität besitzt.

20. Pharmazeutische Zusammensetzung, umfassend ein Polypeptid nach einem der Ansprüche 12-19 und einen physiologisch verträglichen Exzipienten.

21. Antikörper, der an die in Fig. 1 für reife LS-DNase dargestellte Aminosäuresequenz bindet und mit anderen DNase-Proteinen keine Kreuzreaktion eingeht.

22. Antikörper nach Anspruch 21, bei dem es sich um einen monoklonalen Antikörper handelt.

23. Verwendung eines Polypeptids nach einem der Ansprüche 12-19, bei der Herstellung eines Arzneimittels zur Behandlung einer pulmonalen Krankheit oder Störung.

24. Verwendung nach Anspruch 23, wobei es sich bei der Krankheit oder Störung um zystische Fibrose handelt.

25. Verwendung eines Polypeptids nach einem der Ansprüche 12 bis 19 bei der Herstellung eines Arzneimittels zur Behandlung von systemischem Lupus erythematosus.

## Revendications

1. Molécule d'acide nucléique isolée, comprenant une séquence de nucléotides codant une séquence d'acides aminés qui est identique, pour au moins 80 %, à la séquence d'acides aminés présentée sur la figure 1 pour une LS-désoxyribonucléase (LS-DNase) mature et qui possède une activité d'hydrolyse d'ADN.

2. Molécule d'acide nucléique isolée, conforme à la revendication 1, comprenant une séquence de nucléotides codant une séquence d'acides aminés qui est identique, pour au moins 90 %, à la séquence d'acides aminés présentée sur la figure 1 pour une LS-DNase mature et qui possède une activité d'hydrolyse d'ADN.

3. Molécule d'acide nucléique isolée, conforme à la revendication 2, comprenant une séquence de nucléotides codant une séquence d'acides aminés qui est identique, pour au moins 95 %, à la séquence d'acides aminés présentée sur la figure 1 pour une LS-DNase mature et qui possède une activité d'hydrolyse d'ADN.

4. Molécule d'acide nucléique isolée, conforme à la revendication 3, comprenant une séquence de nucléotides codant une séquence d'acides aminés qui est identique, pour au moins 98 %, à la séquence d'acides aminés présentée sur la figure 1 pour une LS-DNase mature et qui possède une activité d'hydrolyse d'ADN.

5. Molécule d'acide nucléique isolée, conforme à la revendication 4, comprenant une séquence de nucléotides codant la séquence d'acides aminés présentée sur la figure 1 pour une LS-DNase mature, qui possède une activité d'hydrolyse d'ADN.

6. Molécule d'acide nucléique isolée, comprenant une séquence de nucléotides codant une séquence d'acides aminés qui ne diffère de la séquence d'acides aminés présentée sur la figure 1 pour une LS-DNase mature que par la substitution d'un acide aminé à un autre en une seule position dans la séquence de la figure 1 et qui possède une activité d'hydrolyse d'ADN.

7. Molécule d'acide nucléique isolée, comprenant une séquence de nucléotides codant une séquence d'acides aminés qui ne diffère de la séquence d'acides aminés présentée sur la figure 1 pour une LS-DNase mature que par la substitution d'un acide aminé à un autre en seulement deux positions dans la séquence de la figure 1 et qui possède une activité d'hydrolyse d'ADN.

8. Vecteur d'expression comprenant une séquence de nucléotides codant la séquence d'acides aminés présentée sur la figure 1 pour une LS-DNase mature, opérationnellement liée à un promoteur reconnu par une cellule hôte transformée au moyen du vecteur.

9. Cellule hôte transformée au moyen d'un vecteur d'expression comprenant une séquence de nucléotides codant la séquence d'acides aminés présentée sur la figure 1 pour une LS-DNase mature.

10. Procédé comportant le fait de transformer une cellule hôte au moyen d'une molécule d'acide nucléique qui code un polypeptide comprenant la séquence d'acides aminés présentée sur la figure 1 pour une LS-DNase mature, et le fait de cultiver cette cellule hôte dans des conditions telles que ledit polypeptide soit produit au sein de la cellule hôte.

11. Procédé de production d'une LS-DNase possédant la séquence d'acides aminés présentée sur la figure 1, lequel procédé comporte :
a) le fait de transformer une cellule contenant un gène de LS-DNase endogène au moyen d'un ADN homologue comprenant un gène amplifiable et une séquence flanquante d'au moins environ 150 paires de bases, qui est homologue à une séquence d'ADN située au sein ou à proximité du gène de LS-DNase endogène, grâce à quoi cet ADN homologue s'intègre dans le génome cellulaire par recombinaison ;
b) le fait de cultiver les cellules dans des conditions qui favorisent sélectivement l'amplification du gène amplifiable, grâce à quoi le gène de LS-DNase est lui aussi amplifié ;
c) et le fait de récupérer ensuite la LS-DNase dans les cellules.

12. Polypeptide isolé comprenant une séquence d'acides aminés qui est identique, pour au moins 80 %, à la séquence d'acides aminés présentée sur la figure 1 pour une LS-DNase mature et qui possède une activité d'hydrolyse d'ADN.

13. Polypeptide isolé conforme à la revendication 12, comprenant une séquence d'acides aminés qui est identique, pour au moins 90 %, à la séquence d'acides aminés présentée sur la figure 1 pour une LS-DNase mature et qui possède une activité d'hydrolyse d'ADN.

14. Polypeptide isolé conforme à la revendication 13, comprenant une séquence d'acides aminés qui est identique, pour au moins 95 %, à la séquence d'acides aminés présentée sur la figure 1 pour une LS-DNase mature et qui possède une activité d'hydrolyse d'ADN.

15. Polypeptide isolé conforme à la revendication 14, comprenant une séquence d'acides aminés qui est identique, pour au moins 98 %, à la séquence d'acides aminés présentée sur la figure 1 pour une LS-DNase mature et qui possède une activité d'hydrolyse d'ADN.

16. Polypeptide isolé conforme à la revendication 15, comprenant la séquence d'acides aminés présentée sur la figure 1 pour une LS-DNase mature, qui possède une activité d'hydrolyse d'ADN.

17. Polypeptide isolé comprenant une séquence d'acides aminés qui ne diffère de la séquence d'acides aminés présentée sur la figure 1 pour une LS-DNase mature que par la substitution d'un acide aminé à un autre en une seule position dans la séquence de la figure 1 et qui possède une activité d'hydrolyse d'ADN.

18. Polypeptide isolé conforme à la revendication 17, dans lequel la substitution d'acide aminé fait apparaître au sein du polypeptide un site de glycosylation qui ne se trouve pas dans la LS-DNase humaine.

19. Polypeptide isolé comprenant une séquence d'acides aminés qui ne diffère de la séquence d'acides aminés présentée sur la figure 1 pour une LS-DNase mature que par la substitution d'un acide aminé à un autre en seulement deux positions dans la séquence de la figure 1 et qui possède une activité d'hydrolyse d'ADN.

20. Composition pharmaceutique comprenant un polypeptide conforme à l'une des revendications 12 à 19, ainsi qu'un excipient physiologiquement admissible.

21. Anticorps qui se lie à la séquence d'acides aminés présentée sur la figure 1 pour une LS-DNase mature et qui ne donne pas lieu à des réactions croisées avec d'autres protéines de type DNase.

22. Anticorps conforme à la revendication 21, qui est un anticorps monoclonal.

23. Emploi d'un polypeptide conforme à l'une des revendications 12 à 19 dans la fabrication d'un médicament destiné au traitement d'une maladie ou d'un trouble pulmonaire.

24. Emploi conforme à la revendication 23, la maladie ou le trou-ble étant la mucoviscidose.

25. Emploi d'un polypeptide conforme à l'une des revendications 12 à 19 dans la fabrication d'un médicament destiné au traitement du lupus érythémateux disséminé.
